(19)

Europäisches
Patentamt

European
Patent Office

Office européen
des brevets

(11)  **EP 1 767 171 B1**

(12)  **EUROPEAN PATENT SPECIFICATION**

(45) Date of publication and mention
of the grant of the patent:
**03.12.2008  Bulletin 2008/49**

(51) Int Cl.:
***A61F 2/34*** (2006.01)

(21) Application number: **06254873.0**

(22) Date of filing: **20.09.2006**

(54) **Prosthetic joints**

Gelenkprothese

Prothèse articulaire

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **23.09.2005  GB 0519490**

(43) Date of publication of application:
**28.03.2007  Bulletin 2007/13**

(73) Proprietor: **BENOIST GIRARD SAS
14201 Hérouville-Saint-Clair Cédex (FR)**

(72) Inventor: **Raugel, Patrick
Ramsey, New Jersey 07446 (US)**

(74) Representative: **Bridge-Butler, Alan James et al
Baron Warren Redfern
19 South End
Kensington
London W8 5BU (GB)**

(56) References cited:
**WO-A-02/43626         WO-A-95/23566
WO-A-97/16138         DE-A1- 19 915 814**

**Description**

This invention relates to prosthetic joints and more particularly to a prosthetic joint which includes a part-spherical femoral bearing head and a flexible bearing element cup or cup bearing liner adapted for use with said bearing head.

Flexible bearing element cups and cup bearing liners are known which include an inner bearing surface which has a first portion which is substantially part-spherical about a hemispherical centre and in which there is a polar bearing area, and a second portion which is relieved with respect to the first portion and which has a radial distance from the hemispherical centre which increases as the angle of rotation about the hemispherical curve increases towards the rim of the cup, the second portion being tangential to the first in a transition portion.

Another construction of this type is shown in PCT Patent Application, publication No. WO 02/43626. The invention in this Application is directed to the curvature of the relieved portion of the cup and the various parameters set out in the Patent are said to create a shape of the non-spherical portion which has the configuration of part of an involute. The concept is defined by regarding the involute as being formed by a thread which is unwould from an imaginary bobbin which is positioned at its circumference on the centre of the sphere which defines the spherical portion of the cup.

Cups of the kind referred to which have a relieved portion with respect to the first hemispherical portion of the cup are employed for various reasons. It is desirable to reduce the contact area of the ball with the cup when reaching extreme angles as this tends to cause friction between them and the relieving alleviates this. Again, the flexibility of the cup may require the relieving to take place and to prevent seizure.

It has been found from experimentation that the amount and shape of the relieving can be varied according to the requirements.

Another consideration is that the fit of the ball in the bearing surface can be closer with a small head ball than with a larger head ball. It is also dependent upon the amount of lubrication in the joint so that amount of relieving must be adjusted appropriately.

The amount of stiffness in the cup or liner effects its deformation.

According to the present invention a prosthetic flexible bearing element cup or cup bearing liner adapted for use with a part-spherical bearing head includes an inner bearing surface which has a first portion which is substantially part-spherical about a hemispherical centre located on a main loading axis, and a second portion at least part of which is relieved with respect to the first portion and which over said relieved part has a radial distance (d) from the hemispherical centre which increases from an inner end towards an outer end as the angle of rotation ($\theta$) about the hemispherical centre increases towards the rim of the cup, characterised in that the distance of the relieved part of the surface of the second portion from a line representing the shape of the outer surface of the part-spherical bearing head when located on said main loading axis and bearing against said first portion of the inner bearing surface at any given radial distance (d) is determined by the formula

$$d = a \left( e^{\theta \cdot \ln b} - 1 \right)$$

where

d          is the radial distance in millimetres between the line representing said shape of the outer surface of the part-spherical bearing head when located on said first portion of the inner bearing surface and with its centre located on said hemispherical centre and the relieved part of the second portion

$\theta$          is the angle in degrees about the hemispherical centre, and

a and b    are values determined by selecting angle an ($\theta_1$) and a distance ($d_1$) at or towards said inner end of the second portion and placing them in said formula, selecting an angle ($\theta_2$) and a distance ($d_2$) at or towards the outer end of second portion adjacent the rim and placing them in the formula and solving the two equations together.

Thus, for varying angles around the length of the second portion, a series of distances (d) can be calculated and which define the shape of the relieving in relation to the line representing said bearing head when in position.

It has been found that the use of the values of a and b in the formula can provide desired shapes of curvature of the relieved portion and which depend upon the angle in degrees about the hemispherical centre and are suitable for use for various requirements. The particular shape for a particular requirement can be found by experimentation.

Preferred successful values of a and b are as set out in the following Table :

| a | b |
| --- | --- |
| 8.2 | 1.001 |
| 1.25 | 1.005 |
| 0.43 | 1.01 |
| 0.305 | 1.012 |
| 0.19 | 1.015 |
| 0.092 | 1.02 |
| 0.046 | 1.025 |
| 0.024 | 1.03 |
| 0.0125 | 1.035 |
| 0.0068 | 1.04 |
| 0.00035 | 1.045 |
| 0.0019 | 1.05 |
| 0.00054 | 1.06 |
| 0.00015 | 1.07 |
| 0.000004 | 1.1 |

Curves with the shapes defined by these numbers have been found to meet most requirements.

Preferably the second portion extends from the main loading axis to the end thereof adjacent its rim.

If desired a transition portion of the inner bearing surface between the first portion and the relieved part of the second portion can be provided. Thus the end of the second portion can overlap the first portion up to the main loading axis, to provide said transition portion.

The inner bearing surface can be formed in a one-piece cup made from, for example, metal, a synthetic plastics material, a combination of synthetic materials and fibres, or a ceramic material.

In an alternative construction the inner bearing surface can be formed on a bearing liner carried in a backing and the liner can be made from metal, a synthetic plastics material, a combination of synthetic material and fibres or a ceramic material, the backing can, for example, be made from metal, synthetic plastics material or a combination of synthetic material and fibres.

In another construction according to the invention the bearing liner can be carried in a second liner in relation to which it can swivel and which is located in fixed or free relationship in the backing to provide a dual mobility cup. This second bearing liner can again be made from metal, synthetic plastics material, a combination of synthetic material and fibres or a ceramic material.

Preferably the radial thickness of the cup or liner is less over the relieved portion than over the part-spherical portion. This assists in determining its flexible qualities.

The construction of the cup or liner and the materials from which it can be made can be as set out in the Applicant's EP Patent No. 0 552 949, EP Patent No. 0 698 382, EP Patent No. 1 208 819, EP Patent No. 1 205 160, UK Patent Application No. 04 22666.8 and UK Patent Application No. 05 07884.5.

Thus, the cup can be made from a combination of PEEK and carbon fibre or any other suitable material.

The invention also includes a prosthetic flexible bearing element, cup or cup bearing liner in combination with a part-spherical bearing head and such a head can be made from any convenient material, for example metal, a synthetic plastics material, a combination of synthetic material and fibres or a ceramic material, or a metal and ceramics composite.

The head can be rigid with a fixing element, for example in the form of a stem or it can be removably attached to such a stem.

The invention can be performed in various ways and some embodiments will now be described by way of example and with reference to the accompanying drawings in which :

Figure 1 is a diagrammatic cross-section of a ball head in a flexible acetabular cup according to the invention;
Figures 2, 3 and 4 are diagrammatic cross-sections of conventional ball and cup constructions which are used to illustrate how the values a and b are determined for use in the formula employed in the invention; and,
Figures 5 to 19 are a series of graphs showing the effect of using values a and b at various angles to produce curved relieved portions of different shapes.

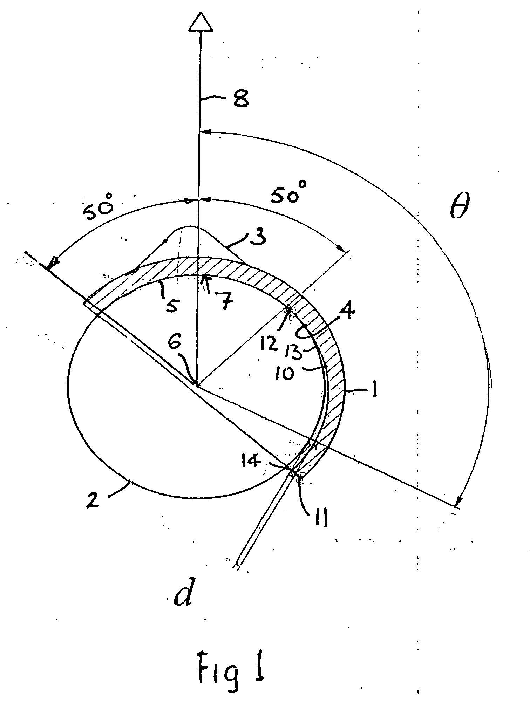

As shown in Figure 1 the cup to which the invention is to be applied is indicated by reference numeral 1 and is for use with a femoral ball head 2, the cup 1 is of the flexible type and can be shaped as set out in EP Patent No. 0 552 949, EP Patent No. 0 698 382, EP Patent No. 1 208 819, EP Patent No. 1 205 160 and UK Patent Application No. 04 22666.8.

A fin 3 of the kind shown in EP Patent No. 1 208 819 can be used. The cup 1 has an inner bearing surface 4 which has a first portion 5 which is substantially part-spherical about a hemispherical centre 6 and in which there is a polar bearing area 7 which is in line with a main loading axis 8. In the construction shown the part-spherical bearing surface 5 extends through about 50 degrees on each side of the main loading axis 8.

A second portion of the inner bearing surface 4 is indicated by reference numeral 10 which extends from the main loading axis 8 to a rim 11 and overlaps the first portion 5 for about 50°. From this point part of the second portion 10 is relieved with respect to the first portion 5 and has a radial distance from the hemispherical centre 6 which increases as the angle of rotation θ about the hemispherical centre 6 from the main loading axis 8 towards the rim 11 of the cup 1 is extended.

Between the first inner surface portion 5 and the second inner surface portion 10 there is a short transition portion 12 provided by the overlap. The inner end of the relieved part of the second inner surface portion adjacent the transition portion 12 is indicated by reference numeral 13 and the outer end adjacent the rim 11 is indicated by reference numeral 14.

As shown in Figure 1 the radial distances of the surface of the second portion 10 from the line representing the shape of the outer surface of the part-spherical bearing head 2 when located on the main loading axis 8 and on said hemispherical centre 6 is indicated by reference letter d.

The distance d determines the amount and shape of the relieving at any given angle θ from the main loading axis 8.

According to the present invention the distance d is determined by the formula

$$d = a\,(e^{\theta \cdot \ln b} - 1)$$

where

d     is the radial distance in millimetres between the line representing said shape of the outer surface of the part-spherical bearing head when located on said first portion of the inner bearing surface and with its centre located on said hemispherical centre and the second portion,

θ     is the angle in degrees about the hemispherical centre,

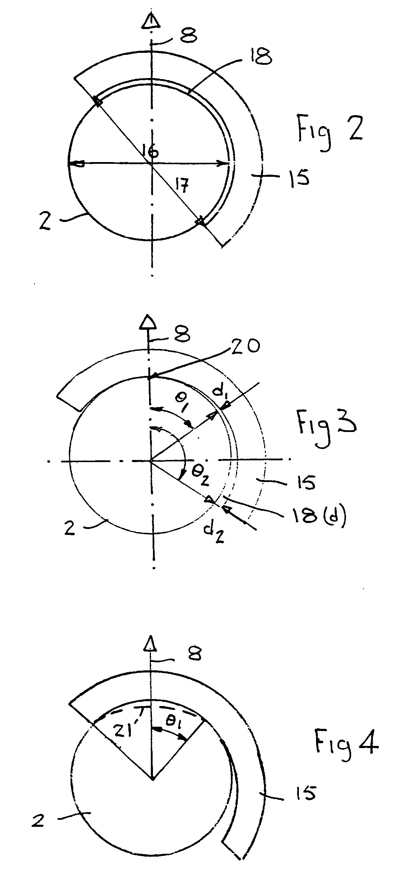

In order to choose the distances d at specific angles θ it is necessary to understand the bearing principals with conventional cups and reference is made to Figures 2, 3 and 4 in which the same reference numerals are used to indicated similar parts to those shown in Figure 1.

When a conventional femoral ball head 2 is placed in a conventional cup, indicated by reference numeral 15 in Figure 2, in order to allow movement of the head in the cup the diameter 16 of the head 2 has to be smaller than the inner diameter 17 of the cup 15. This is necessary because the surfaces of the head and cup are never perfect. As a consequence of which there is a clearance 18 which is commonly defined as the radial clearance which is :

$$r \qquad \text{radial clearance = radius of the cup - radius of the head}$$

The value of the radial clearance depends on the materials used for the head and the cup. It is usually around 50 μ to 100 μ m for hard bearing couples (metal head into metal cup or ceramic into ceramic cup) and 150 μm to 300 μm for a soft bearing couple (metal or ceramic head into a polyethylene cup). If a composite material is used for the cup which is articulating against a ceramic head, the radial clearance can also be 150 μm to 300 μm.

When a load is applied and the head and the cup are both made of non-deformable material, the head will move along the main loading axis 8 and come into contact with the inner surface of the cup, as shown in Figure 3. When this occurs there is only one point contact, indicated by reference numeral 20, with the inner surface of the cup 15. The distance d, indicated by reference numeral 18, between the head and the cup is increasing progressively about an angle θ from the main loading axis 8, as shown in Figure 1.

If the head and the cup were considered as being formed from elastic material (usually polyethylene) for the cup and steel for the head then a deformation will happen predominantly on the softer material. The softer the material the higher the deformation under the same loading condition. Figure 4 shows the deformation of the cup and the broken line 21 shows the shape of the cup prior to the deformation. In this case the contact between the head and the cup forms a sector on which the half-angle is $\theta_1$.

This angle $\theta_1$ can be determined by experimentation or calculation, thus for a couple of bearing materials the angle of the contact sector under defined loading conditions can be predicted. With the flexible cup, the bearing portion will function similarly to a conventional cup. Therefore it is possible to predict the contact sector between the head and the cup and find the angle $\theta_1$ corresponding to the end of the deformation of the softer material. In the example shown in Figure 1 this angle is 50 degrees. It is now necessary to determine the distance $d_1$ at the angle $\theta_1$. This is done by returning to the non-deformable conditions shown in Figure 3 and measuring the distance $d_1$ at said angle $\theta_1$. This distance can also be easily calculated. In the example shown in Figure 1 the distance can be 0.09 mm. This means that the curve between $\theta = 0$ degrees and $\theta = 50$ degrees will be similar to the curve of a conventional cup with a spherical inner cavity.

The second specific angle $\theta_2$, as shown in Figure 3, is taken at the end of the cup when it is placed in the standard position. At this angle distance $d_2$ is again required between the end of the cup adjacent the rim so that it accommodates its predictable deformity of the reamed host bone and also accommodates the natural deformation of the acetabulum during normal gait cycle. These figures come from geometrical analysis of the acetabulum after reaming and strain analysis during the gait.

In the example shown in Figure 3 the distance $d_2$ at the angle $\theta_2$ is 1.2 mm. Thus, the curvature generated with the function would pass through these two specific points defined.

If the first angle $\theta_1$ is taken at 50 degrees and the second angle $\theta_2$ at 130 degrees and the distance $d_1$ at the first angle is 0.09 mm and the distance $d_2$ at 1.2 mm then the two equations are set out as follows :

$$0.09 = a(e^{\,50 \ln b} - 1) \quad \text{and} \quad 1.20 = a(e^{\,130 \ln b} - 1)$$

From the two equations it is possible to determine the following values for **a** and **b** :

$$a \approx 0.02689425 \quad \text{and} \quad b \approx 1.0298232$$

The equation of the relieving curve is then established and can be written :

$$d = 0.02689425(e^{\,\theta \ln 1.0298232} - 1)$$

The distance $d_1$ can be between 0.050 mm to 0.500 mm and the distance $d_2$ can be between 0.400 mm and 5,000 mm.

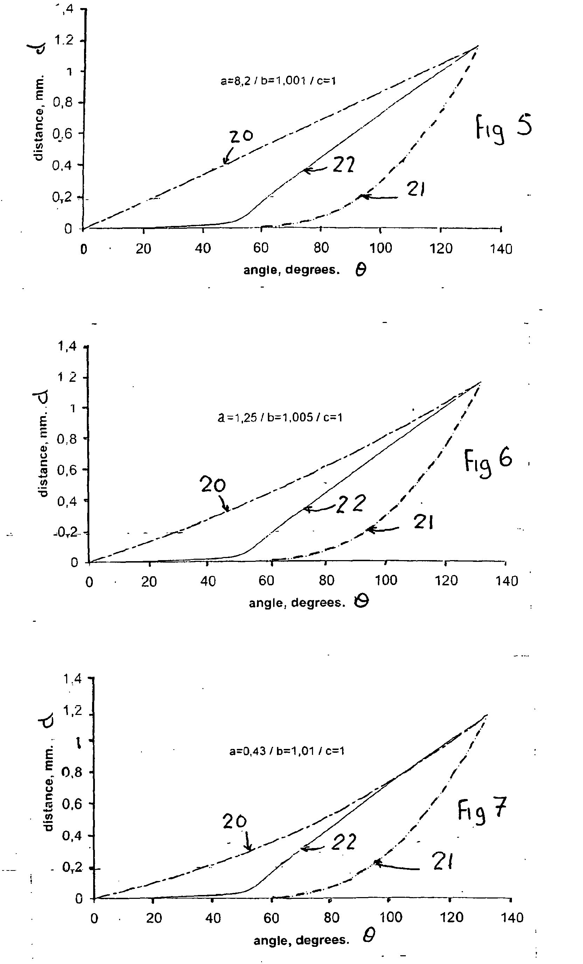

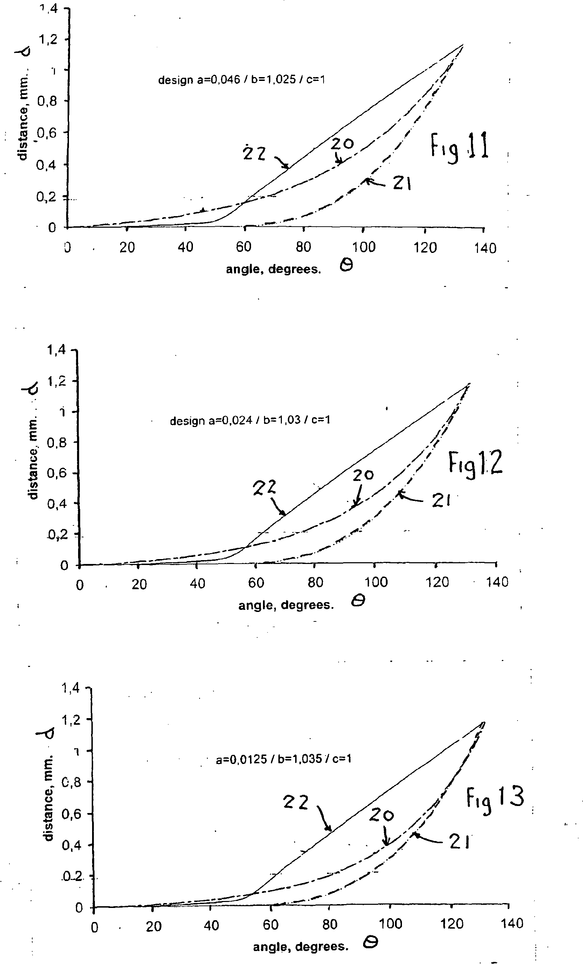

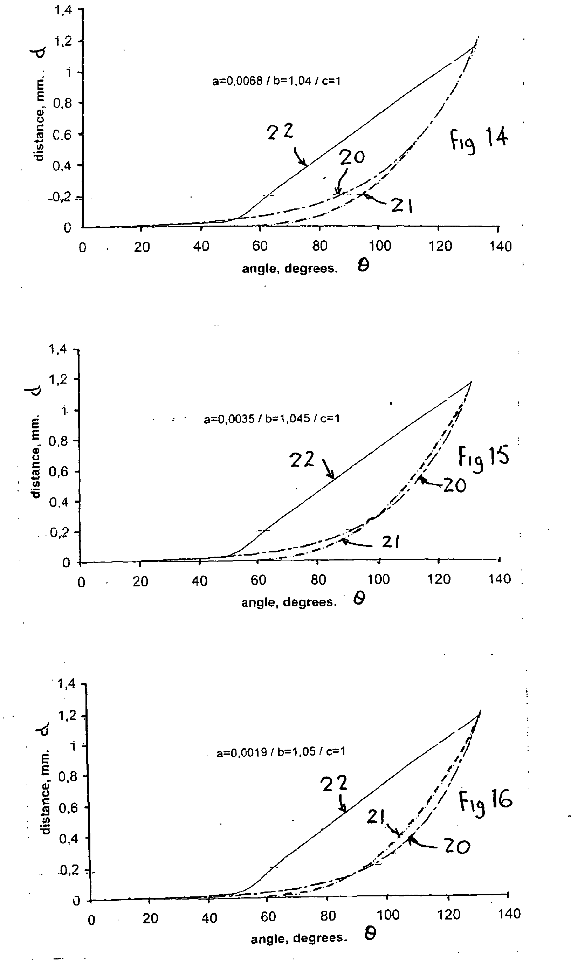

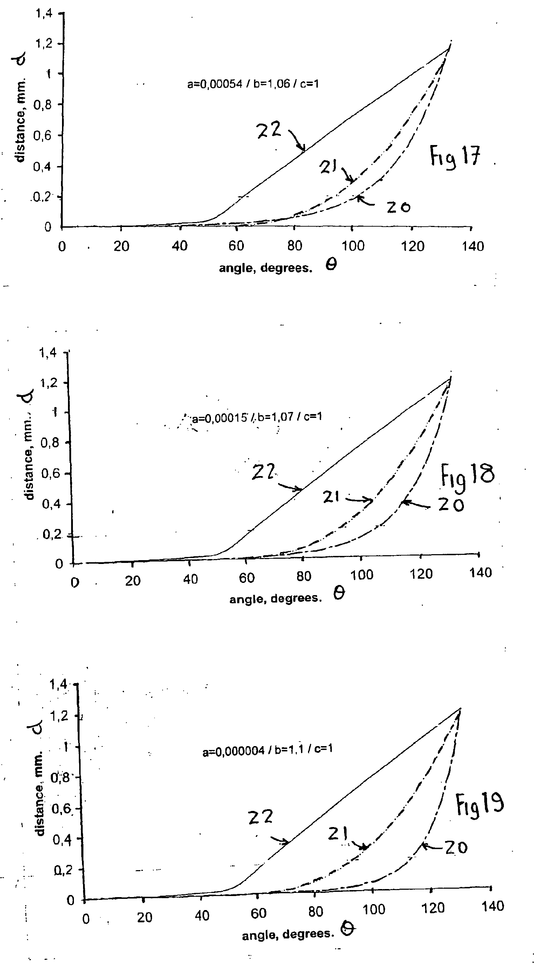

The graphs shown in Figures 5 to 19 show the distance d in millimetres for the determined degree $\theta$ from the main loading axis 8.

In the graph shown in Figure 5 the a and b values are indicated and the broken chain line 20 shows the distance d for the various angles $\theta$. It will be seen that the line is substantially straight and the relieving commences from the main loading axis 8. This type of relieving is suitable for use with flexible cups which are relatively stiff. In this arrangement the first part-spherical portion 5 only extends over a curve of about 50 degrees to one side of the main bearing axis 8.

For comparison the graph also shows the distance d for various angles $\theta$ for a construction made according to the information set out in WO 02/43626 and this is indicated by broken line 21 and solid line 22 shows the distances for relieving as set out in EP Patent No. 0 552 949.

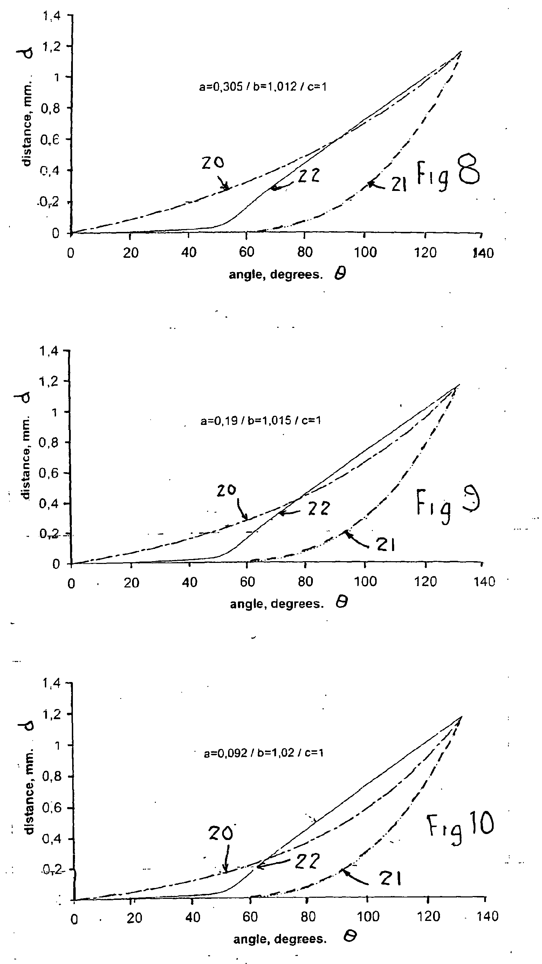

Graphs shown in Figures 6, 7 and 8 show variations on the shape as compared with the graph shown in Figure 5 and in Figure 9 the dimensions produced by the formula still provide for the commencement of relieving in the area of the main bearing axis 8.

From graph 10 to graph 13 the dimensions become smaller as the angle moves away from the main bearing axis 8 until graph 14 where it will be seen that there is little movement until about 50 degrees from the main bearing angle. The movement then is gradual so that there is, in effect, a transition portion between about 50 degrees and 60 degrees.

From graphs 15 to 19 there is again only a small amount of movement and from graph 19 there is little movement away from the part-spherical surface of the first portion of the inner bearing surface until the angle is about 80 degrees.

The later curves shown from about graphs 14 to 19 are more suitable for cups which are more flexible.

The invention is particularly designed to produce cups in which a particular effect is required to reduce the possibility of seizure or heavy wear.

A method of making a prosthetic cup of the kind as set forth is now described.

The values obtained with the function described above in the present invention need to be added to the femoral head radius in order to generate the inner shape of the cup. This is a basic transformation done with convention Computer Added Design (CAD) software, as well as the transformation of the function in a series of co-ordinates of points that are used to program a Computerised Numerical Control (CNC) lathe. The transformation usually needs to tolerate an approximation between the theoretical curve and actual curve that is used for the machining. A 1 $\mu$m approximation allows to generate a curve with an acceptable number of points.

The construction of an acetabular cup including an inner portion following the curve described in the present invention requires to position the inner portion relative to the outer shape of the cup. The objective is to maintain the centre of rotation of the prosthetic head as close as possible to the natural centre of rotation, therefore, the centre of the femoral head is usually close to the centre of the outer part-spherical portion of the cup. The application of this rule to the present invention will result in an acetabular prosthesis with a variable cup wall thickness. In the present construction the thickest wall is preferably located in the bearing portion and the thinner wall is positioned near the opened portion of the cup. This thickness distribution allows to stiffen the portion where deformation needs to be avoided and soften the portion where deformation is expected.

## Claims

1. A prosthetic flexible bearing element cup or cup bearing liner (1) adapted for use with a part-spherical bearing head (2) which includes an inner bearing surface (4) which has a first portion (5) which is substantially part-spherical about a hemispherical centre (6) located on a main loading axis (8), and a second portion (10) at least part of which is relieved with respect to the first portion (5) and which over said relieved part has a radial distance (d) from the hemispherical centre (6) which increases from an inner end towards an outer end as the angle of rotation ($\theta$) about the hemispherical centre increases towards the rim (11) of the cup, **characterised in that** the distance of the relieved part of the surface of the second portion (10) from a line representing the shape of the outer surface of the part-spherical bearing head (2) when located on said main loading axis (8) and bearing against said first portion (5) of the inner bearing surface (4) at any given radial distance (d) is determined by the formula

$$d = a \left( e^{\,\theta \cdot \ln b} - 1 \right)$$

where

d is the radial distance in millimetres between the line representing said shape of the outer surface of the part-spherical bearing head (2) when located on said first portion (5) of the inner bearing surface (4) and with its centre located on said hemispherical centre (6) and the relieved part of the second portion (10)
$\theta$ is the angle in degrees about the hemispherical centre (6), and
a and b are values determined by selecting angle ($\theta_1$) and a distance ($d_1$) at or towards said inner end of the second portion (10) and placing them in said formula, selecting an angle ($\theta_2$) and a distance ($d_2$) at or towards the outer end of second portion (10) adjacent the rim (11) and placing them in the formula and solving the two equations together.

2. A prosthetic flexible bearing element cup or cup bearing liner as claimed in claim 1 **characterised in that** the values of a and b are selected from the following Table :

| a | b |
|---|---|
| 8.2 | 1.001 |
| 1.25 | 1.005 |
| 0.43 | 1.01 |
| 0.305 | 1.012 |
| 0.19 | 1.015 |
| 0.092 | 1.02 |
| 0.046 | 1.025 |

(continued)

| a | b |
|---|---|
| 0.024 | 1.03 |
| 0.0125 | 1.035 |
| 0.0068 | 1.04 |
| 0.00035 | 1.045 |
| 0.0019 | 1.05 |
| 0.00054 | 1.06 |
| 0.00015 | 1.07 |
| 0.000004 | 1.1 |

3. A prosthetic flexible bearing element cup or cup bearing liner as claimed in claim 1 or claim 2 **characterised in that** the second portion (10) extends from the main loading axis (8) to the end thereof adjacent its rim (11).

4. A prosthetic flexible bearing element cup or cup bearing liner as claimed in any one of claims 1 to 3 **characterised in that** a transition portion (12) of the inner bearing surface between the first portion (5) and the relieved part of the second portion (10) is provided.

5. A prosthetic flexible bearing element cup or cup bearing liner as claimed in claim 4 **characterised in that** the end of the second portion (10) overlaps the first portion (5) up to the main loading axis (8), to provide said transition portion (12).

6. A prosthetic flexible bearing element cup or cup bearing liner as claimed in any one of preceding claims 1 to 5 **characterised in that** the inner bearing surface (4) is formed in a one-piece cup.

7. A prosthetic flexible bearing element cup or cup bearing liner as claimed in claim 6 **characterised in that** the one-piece cup is made from metal, a synthetic plastics material, a combination of synthetic materials and fibres, or a ceramic material.

8. A prosthetic flexible bearing element cup or cup bearing liner as claimed in any one of preceding claims 1 to 5 **characterised in that** the inner bearing surface (4) is formed on a bearing liner carried in a backing.

9. A prosthetic flexible bearing element cup or cup bearing liner as claimed in claim 8 **characterised in that** the liner is made from metal, a synthetic plastics material, a combination of synthetic material and fibres or a ceramic material.

10. A prosthetic flexible bearing element cup or cup bearing liner as claimed in claim 8 or claim 9 **characterised in that** the backing is made from metal, synthetic plastics material or a combination of synthetic material and fibres.

11. A prosthetic flexible bearing element cup or cup bearing liner as claimed in claim 8 or claim 9 **characterised in that** the bearing liner is carried in a second liner in relation to which it can swivel and which is located in fixed or free relationship in the backing to provide a dual mobility cup.

12. A prosthetic flexible bearing element cup or cup bearing liner as claimed in claim 11 **characterised in that** the second bearing liner is made from metal, synthetic plastics material, a combination of synthetic material and fibres or a ceramic material.

13. A prosthetic flexible bearing element cup or cup bearing liner as claimed in any one of preceding claims 1 to 12 **characterised in that** the radial thickness of the cup or liner (1) is less over the relieved portion than over the part-spherical portion (5).

14. A prosthetic flexible bearing element cup or cup bearing liner as claimed in any one of the preceding claims 1 to 13 **characterised in that** the cup (1) is made from a combination of PEEK and carbon fibre.

15. A prosthetic flexible bearing element cup or cup bearing liner as claimed in any one of the preceding claims 1 to 14 in combination with a part-spherical bearing head (2).

**16.** A prosthetic flexible bearing element cup or cup bearing liner as claimed in claim 15 in combination with a part-spherical bearing head (2) which is made from metal, a synthetic plastics material, a combination of synthetic material and fibres or a ceramic material, or a metal and ceramics composite.

**17.** A prosthetic flexible bearing element cup or cup bearing liner in combination with a head (2) as claimed in claim 15 or claim 16 **characterised in that** the head (2) is rigid with a fixing element.

**18.** A prosthetic flexible bearing element cup or cup bearing liner as claimed in claim 17 **characterised in that** the fixing element is in the form of a stem or it can be removably attached to such a stem.

**19.** A prosthetic flexible bearing element cup or cup bearing liner as claimed in claim 16 in which the head (2) is removably attached to a stem.

**Patentansprüche**

**1.** Prothetische flexible Lagerelementpfanne oder Pfannenlagerauskleidung (1), angepasst zur Verwendung mit einem teilsphärischen Lagerkopf (2), die eine innere Lageroberfläche (4) einschließt, welche einen ersten Teil (5) aufweist, der im Wesentlichen teilsphärisch um einen auf einer Hauptlastachse (8) angeordnete Halbkugelmittelpunkt (6) angeordnet ist, sowie einen zweiten Teil (10), von dem mindestens ein Teil in Bezug zum ersten Teil (5) freigespart ist und der über den freigesparten Teil vom Halbkugelmittelpunkt (6) einen radialen Abstand (d) aufweist, welcher von einem inneren Ende aus in Richtung eines äußeren Endes zunimmt, während der Drehwinkel (θ) um den Halbkugelmittelpunkt zum Rand (11) der Pfanne hin zunimmt, **dadurch gekennzeichnet, dass** der Abstand des freigesparten Teils der Oberfläche des zweiten Teils (10) von einer Linie, welche die Form der äußeren Oberfläche des teilsphärischen Lagerkopfs (2) darstellt, wenn er auf der Hauptlastachse (8) angeordnet ist und gegen den ersten Teil (5) der inneren Lageroberfläche (4) anliegt, bei einem beliebigen gegebenen radialen Abstand (d) bestimmt wird durch die Formel

$$d = a\,(e^{\theta.\ln b} - 1)$$

wobei

d der radiale Abstand in Millimetern zwischen der Linie, welche die Form der äußeren Oberfläche des teilsphärischen Lagerkopfs (2) darstellt, wenn er auf dem ersten Teil (5) der inneren Lageroberfläche (4) angeordnet und mit seinem Mittelpunkt auf dem Halbkugelmittelpunkt (6) angeordnet ist, und dem freigesparten Teil des zweiten Teils (10) ist,
θ der Winkel in Grad um den Halbkugelmittelpunkt (6) ist, und
a und b Werte sind, die bestimmt werden, indem man einen Winkel ($\theta_1$) und einen Abstand ($d_1$) an oder zu dem inneren Ende des zweiten Teils (10) hin auswählt und in die Formel einsetzt, einen Winkel ($\theta_2$) und einen Abstand ($d_2$) an oder zu dem zum Rand (11) benachbarten äußeren Ende des zweiten Teils (10) hin auswählt und in die Formel einsetzt, und indem man die beiden Gleichungen zusammen auflöst.

**2.** Prothetische flexible Lagerelementpfanne oder Pfannenlagerauskleidung nach Anspruch 1, **dadurch gekennzeichnet, dass** die Werte von a und b aus der folgenden Tabelle ausgewählt werden:

| a | b |
|---|---|
| 8,2 | 1,001 |
| 1,25 | 1,005 |
| 0,43 | 1,01 |
| 0, 305 | 1,012 |
| 0, 19 | 1,015 |
| 0,092 | 1,02 |
| 0,046 | 1,025 |
| 0,024 | 1,03 |

(fortgesetzt)

| a | b |
|---|---|
| 0,0125 | 1,035 |
| 0,0068 | 1,04 |
| 0,00035 | 1,045 |
| 0,0019 | 1,05 |
| 0,00054 | 1,06 |
| 0,00015 | 1,07 |
| 0,000004 | 1, 1 |

3. Prothetische flexible Lagerelementpfanne oder Pfannenlagerauskleidung nach Anspruch 1 oder Anspruch 2, **dadurch gekennzeichnet, dass** sich der zweite Teil (10) von der Hauptlastachse (8) bis zu seinem Ende erstreckt, das zu ihrem Rand (11) benachbart ist.

4. Prothetische flexible Lagerelementpfanne oder Pfannenlagerauskleidung nach einem der Ansprüche 1 bis 3, **dadurch gekennzeichnet, dass** zwischen dem ersten Teil (5) und dem freigesparten Teil des zweiten Teils (10) ein Übergangsteil (12) der inneren Lageroberfläche vorgesehen ist.

5. Prothetische flexible Lagerelementpfanne oder Pfannenlagerauskleidung nach Anspruch 4, **dadurch gekennzeichnet, dass** des Ende des zweiten Teil (10) den ersten Teil (5) bis hin zur Hauptlastachse (8) überlappt, um den übergangsteil (12) bereitzustellen.

6. Prothetische flexible Lagerelementpfanne oder Pfannenlagerauskleidung nach einem der vorangehenden Ansprüche 1 bis 5, **dadurch gekennzeichnet, dass** die innere Lageroberfläche (4) in einer einteiligen Pfanne ausgebildet ist.

7. Prothetische flexible Lagerelementpfanne oder Pfannenlagerauskleidung nach Anspruch 6, **dadurch gekennzeichnet, dass** die einteilige Pfanne aus Metall, einem Kunststoffmaterial, einer Kombination von Kunststoffmaterialien und Fasern oder einem Keramikmaterial hergestellt ist.

8. Prothetische flexible Lagerelementpfanne oder Pfannenlagerauskleidung nach einem der vorangehenden Ansprüche 1 bis 5, **dadurch gekennzeichnet, dass** die innere Lageroberfläche (4) auf einer in einem Träger aufgenommenen Lagerauskleidung ausgebildet ist.

9. Prothetische flexible Lagerelementpfanne oder Pfannenlagerauskleidung nach Anspruch 8, **dadurch gekennzeichnet, dass** die Auskleidung aus Metall, einem Kunststoffmaterial, einer Kombination von Kunststoffmaterial und Fasern oder einem Keramikmaterial hergestellt ist.

10. Prothetische flexible Lagerelementpfanne oder Pfannenlagerauskleidung nach Anspruch 8 oder Anspruch 9, **dadurch gekennzeichnet, dass** der Träger aus Metall, Kunststoffmaterial oder einer Kombination von Kunststoffmaterial und Fasern hergestellt ist.

11. Prothetische flexible Lagerelementpfanne oder Pfannenlagerauskleidung nach Anspruch 8 oder Anspruch 9, **dadurch gekennzeichnet, dass** die Lagerauskleidung in einer zweiten Auskleidung aufgenommen ist, in Bezug zu der sie sich drehen kann und die in fester oder freier Beziehung in dem Träger angeordnet ist, um eine Pfanne mit Doppelbeweglichkeit bereitzustellen.

12. Prothetische flexible Lagerelementpfanne oder Pfannenlagerauskleidung nach Anspruch 11, **dadurch gekennzeichnet, dass** die zweite Lagerauskleidung aus Metall, Kunststoffmaterial, einer Kombination von Kunststoffmaterial und Fasern oder einem Keramikmaterial hergestellt ist.

13. Prothetische flexible Lagerelementpfanne oder Pfannenlagerauskleidung nach einem der vorangehenden Ansprüche 1 bis 12, **dadurch gekennzeichnet, dass** die radiale Dicke der Pfanne oder Auskleidung (1) über dem freigesparten Teil kleiner als über dem teilsphärischen Teil (5) ist.

14. Prothetische flexible Lagerelementpfanne oder Pfannenlagerauskleidung nach einem der vorangehenden Ansprüche 1 bis 13, **dadurch gekennzeichnet, dass** die Pfanne (1) aus einer Kombination von PEEK und Kohlenstofffasern hergestellt ist.

15. Prothetische flexible Lagerelementpfanne oder Pfannenlagerauskleidung nach einem der vorangehenden Ansprüche 1 bis 14 in Kombination mit einen teilsphärischen Lagerkopf (2).

16. Prothetische flexible Lagerelementpfanne oder Pfannenlagerauskleidung nach Anspruch 15 in Kombination mit einem teilsphärischen Lagerkopf (2), der aus Metall, einem Kunststoffmaterial, einer Kombination von Kunststoffmaterial und Fasern oder einem Keramikmaterial oder einem Metall- und Keramik-Verbundwerkstoff hergestellt ist.

17. Prothetische flexible Lagerelementpfanne oder Pfannenlagerauskleidung in Kombination mit einem Kopf (2) nach Anspruch 15 oder Anspruch 16, **dadurch gekennzeichnet, dass** der Kopf (2) mit einem Befestigungselement starr verbunden ist.

18. Prothetische flexible Lagerelementpfanne oder Pfannenlagerauskleidung nach Anspruch 17, **dadurch gekennzeichnet, dass** das Befestigungselement in Form eines Schaftes vorliegt oder dass es abnehmbar an einem solchen Schaft befestigt werden kann.

19. Prothetische flexible Lagerelementpfanne oder Pfannenlagerauskleidung nach Anspruch 16, bei welcher der Kopf (2) abnehmbar an einem Schaft befestigt ist.

**Revendications**

1. Coupelle ou chemisage de support de coupelle d'élément de support souple prothétique (1) adapté pour l'utilisation avec une tête de support partiellement sphérique (2) qui comprend une surface de portée intérieure (4) qui comporte une première partie (5) qui est sensiblement partiellement sphérique autour d'un centre hémisphérique (6) situé sur un axe de charge principal (8), et une deuxième partie (10) dont au moins une partie est relâchée vis-à-vis de la première partie (5), et qui, sur ladite partie relâchée, a une distance radiale (d) par rapport au centre hémisphérique (6) qui augmente d'une extrémité intérieure à une extrémité extérieure lorsque l'angle de rotation (9) autour du centre hémisphérique augmente vers le rebord (11) de la coupelle, **caractérisé en ce que** la distance de la partie relâchée de la surface de la deuxième partie par rapport à une ligne représentant la forme de la surface extérieure de la tête de support partiellement sphérique (2) lorsqu'elle est située sur ledit axe de charge principal (8) et portant contre ladite première partie (5) de la surface de portée intérieure (4) à n'importe quelle distance radiale donnée (d) est déterminée par la formule :

$$d = a(e^{\theta \cdot \ln b} - 1)$$

où :

d est la distance radiale en millimètres entre la ligne représentant ladite forme de la surface extérieure de la tête de support partiellement sphérique (2) lorsqu'elle est située sur ladite première partie (5) de la surface de portée intérieure (4) et avec son centre situé sur ledit centre hémisphérique (6) et la partie relâchée de la deuxième partie (10),
θ est l'angle en degrés autour du centre hémisphérique (6), et
a et b sont des valeurs déterminées par la sélection d'un angle ($\theta_1$) et d'une distance ($d_1$) à ou vers ladite extrémité intérieure de la deuxième partie (10) et l'inclusion de ceux-ci dans ladite formule, la sélection d'un angle ($\theta_2$) et d'une distance ($d_2$) à ou vers l'extrémité extérieure de la deuxième partie (10) au voisinage du rebord (11) et l'inclusion de ceux-ci dans la formule et la résolution des deux équations ensemble.

2. Coupelle ou chemisage de support de coupelle d'élément de support souple prothétique selon la revendication 1, **caractérisé en ce que** les valeurs de a et de b sont sélectionnées à partir du tableau suivant :

| a | b |
|---|---|
| 8,2 | 1,001 |

(suite)

| a | b |
|---|---|
| 1,25 | 1,005 |
| 0,43 | 1,01 |
| 0,305 | 1,012 |
| 0,19 | 1,015 |
| 0,092 | 1,02 |
| 0,046 | 1,025 |
| 0,024 | 1,03 |
| 0,0125 | 1,035 |
| 0,0068 | 1,04 |
| 0,00035 | 1,045 |
| 0,0019 | 1,05 |
| 0,00054 | 1,06 |
| 0,00015 | 1,07 |
| 0,000004 | 1,1 |

3. Coupelle ou chemisage de support de coupelle d'élément de support souple prothétique selon la revendication 1 ou la revendication 2, **caractérisé en ce que** la deuxième partie (10) s'étend de l'axe de charge principal (8) à l'extrémité de celle-ci adjacente à sa bordure (11).

4. Coupelle ou chemisage de support de coupelle d'élément de support souple prothétique selon l'une quelconque des revendications 1 à 3, **caractérisé en ce qu'**une partie de transition (12) de la surface de portée intérieure entre la première partie (5) et la partie relâchée de la deuxième partie (10) est présente.

5. Coupelle ou chemisage de support de coupelle d'élément de support souple prothétique selon la revendication 4, **caractérisé en ce que** l'extrémité de la deuxième partie (10) chevauche la première partie (5) jusqu'à l'axe de charge principal (8), de façon à constituer ladite partie de transition (12).

6. Coupelle ou chemisage de support de coupelle d'élément de support souple prothétique selon l'une quelconque des revendications 1 à 5 qui précèdent, **caractérisé en ce que** la surface de portée intérieure (4) est formée sous la forme d'une coupelle d'une seule pièce.

7. Coupelle ou chemisage de support de coupelle d'élément de support souple prothétique selon la revendication 6, **caractérisé en ce que** la coupelle d'une seule pièce est réalisée en métal, en une matière plastique synthétique, en une combinaison de matériaux synthétiques et de fibres, ou en un matériau céramique.

8. Coupelle ou chemisage de support de coupelle d'élément de support souple prothétique selon l'une quelconque des revendications 1 à 5 qui précèdent, **caractérisé en ce que** la surface de portée intérieure (4) est formée sur un chemisage de support porté dans un revêtement de revers.

9. Coupelle ou chemisage de support de coupelle d'élément de support souple prothétique selon la revendication 8, **caractérisé en ce que** le chemisage est réalisé en métal, en une matière plastique synthétique, en une combinaison de matériau synthétique et de fibres ou en un matériau céramique.

10. Coupelle ou chemisage de support de coupelle d'élément de support souple prothétique selon la revendication 8 ou la revendication 9, **caractérisé en ce que** le revêtement de revers est réalisé en métal, en une matière plastique synthétique ou en une combinaison d'un matériau synthétique et de fibres.

11. Coupelle ou chemisage de support de coupelle d'élément de support souple prothétique selon la revendication 8 ou la revendication 9, **caractérisé en ce que** le chemisage de support est porté dans un deuxième chemisage par rapport auquel il peut pivoter et qui est disposé en relation fixe ou libre dans le revêtement de revers de façon à constituer une coupelle à double mobilité.

12. Coupelle ou chemisage de support de coupelle d'élément de support souple prothétique selon la revendication 11, **caractérisé en ce que** le deuxième chemisage de support est réalisé en métal, en une matière plastique synthétique, en une combinaison de matière synthétique et de fibres ou en un matériau céramique.

13. Coupelle ou chemisage de support de coupelle d'élément de support souple prothétique selon l'une quelconque des revendications 1 à 12 qui précèdent, **caractérisé en ce que** l'épaisseur radiale de la coupelle ou du chemisage (1) est plus petite sur la partie relâchée que sur la partie partiellement sphérique (5).

14. Coupelle ou chemisage de support de coupelle d'élément de support souple prothétique selon l'une quelconque des revendications 1 à 13 qui précèdent, **caractérisé en ce que** la coupelle (1) est réalisée en une combinaison de polyéther-éther-cétone et de fibre de carbone.

15. Coupelle ou chemisage de support de coupelle d'élément de support souple prothétique selon l'une quelconque des revendications 1 à 14 qui précèdent en combinaison avec une tête de support partiellement sphérique (2).

16. Coupelle ou chemisage de support de coupelle d'élément de support souple prothétique selon la revendication 15 en combinaison avec une tête de support partiellement sphérique (2) qui est réalisée en métal, en une matière plastique synthétique, en une combinaison de matériau synthétique et de fibres ou en un matériau céramique, ou en un composite de métal et de céramique.

17. Coupelle ou chemisage de support de coupelle d'élément de support souple prothétique en combinaison avec une tête (2) selon la revendication 15 ou la revendication 16, **caractérisé en ce que** la tête (2) est rigide avec un élément de fixation.

18. Coupelle ou chemisage de support de coupelle d'élément de support souple prothétique selon la revendication 17, **caractérisé en ce que** l'élément de fixation se présente sous la forme d'une tige ou **en ce qu'**il peut être fixé de façon amovible à cette tige.

19. Coupelle ou chemisage de support de coupelle d'élément de support souple prothétique selon la revendication 16, dans lequel la tête (2) est fixée de façon amovible à une tige.

Fig 1

Fig 2

Fig 3

Fig 4

a=8,2 / b=1,001 / c=1

Fig 5

a=1,25 / b=1,005 / c=1

Fig 6

a=0,43 / b=1,01 / c=1

Fig 7

a=0,305 / b=1,012 / c=1

20

22

21 Fig 8

distance, mm.  d

angle, degrees.  θ

a=0,19 / b=1,015 / c=1

20

22

21

Fig 9

distance, mm.  d

angle, degrees.  θ

a=0,092 / b=1,02 / c=1

20

22

21

Fig 10

distance, mm.  d

angle, degrees.  θ

Fig 11
design a=0,046 / b=1,025 / c=1

Fig 12
design a=0,024 / b=1,03 / c=1

Fig 13
a=0,0125 / b=1,035 / c=1

a=0,0068 / b=1,04 / c=1

Fig 14

a=0,0035 / b=1,045 / c=1

Fig 15

a=0,0019 / b=1,05 / c=1

Fig 16

Fig 17

a=0,00054 / b=1,06 / c=1

22  21  20

distance, mm. d

angle, degrees. θ

Fig 18

a=0,00015 / b=1,07 / c=1

22  21  20

distance, mm. d

angle, degrees. θ

Fig 19

a=0,000004 / b=1,1 / c=1

22  21  20

distance, mm. d

angle, degrees. θ

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0243626 A
- EP 0552949 A
- EP 0698382 A
- EP 1208819 A
- EP 1205160 A
- GB 0422666 A
- GB 0507884 A